# FASCICULE DE BREVET EUROPEEN

(11) **EP 2 308 532 B1**
(45) Date de publication et mention de la délivrance du brevet: **11.06.2014**
(21) Numéro de dépôt: 10184229.2
(22) Date de dépôt: 11.02.2003
(51) Int. Cl.: A61M 5/50, A61M 5/31

(54) **Dispositif de support de sécurité pour une seringue et ensemble d'un tel dispositif et d'une seringue**
Sicherheitsvorrichtung für eine Spritze und Anordnung bestehend aus dieser Vorrichtung und einer Spritze
Security support device for a syringe and assambly of such a device and a syringe

(30) Priorité: 11.02.2002 FR 0201643
(43) Date de publication de la demande: 13.04.2011
(62) Demande divisionnaire de: 03739519.1
(73) Titulaire: SANOFI, 75008 Paris (FR)
(72) Inventeur: Chevallier, Stéphane, 77165, Saint-Soupplets (France) (FR)
(74) Mandataire: Intès, Didier Gérard André

(56) Documents cités:
- WO-A-01/85239
- FR-A- 2 801 795
- US-B1- 6 319 234

## Description

La présente invention concerne un dispositif de support de sécurité pour une seringue, comprenant un fourreau de support pour le corps d'une seringue et des moyens de sécurité qui comprennent un fourreau intérieur susceptible de coulisser par rapport au dit fourreau de support, entre une position escamotée d'attente, dans laquelle ledit fourreau intérieur est sensiblement escamoté à l'intérieur dudit fourreau de support, et une position active de protection, dans laquelle ledit fourreau intérieur dépasse dudit fourreau de support.

L'invention concerne également un ensemble constitué d'un tel dispositif et d'une seringue.

Un tel dispositif permet de protéger l'utilisateur contre tout risque de piqûre accidentelle après utilisation de la seringue et donc d'une contamination éventuelle, en entourant l'aiguille de la seringue par le fourreau intérieur, qui est sollicité vers sa position active de protection par des moyens de poussée tels qu'un ressort.

On connaît de tels dispositifs et ensembles qui permettent de protéger l'aiguille par déplacement d'un fourreau intérieur dans un fourreau de support.

Le document FR 2 801 795 décrit un ensemble dans lequel la sortie du fourreau intérieur peut être déclenchée automatiquement par des moyens de poussée (en l'espèce, par des moyens élastiques) pour adopter en fin d'injection, une position active de protection autour de l'aiguille. Cependant, le premier contact du fourreau intérieur avec la chair du patient peut être quelque peu inattendu et surprendre le patient, d'autant que le contact s'effectue de manière dynamique. Par la suite, une légère pression, due à la poussée exercée par les moyens élastiques sur le fourreau intérieur jusqu'à son extraction totale hors du fourreau de support, peut être ressentie par le patient, jusqu'à ce que l'aiguille soit complètement retirée de sa chair. Toutefois, cette pression est statique, donc sans risque contondant pour le patient.

Dans ce type de dispositif, les moyens élastiques sont généralement tarés pour limiter l'intensité de l'appui en provoquant un coulissement délicat du fourreau intérieur hors du fourreau de support qui ne heurte pas la chair du patient. Ainsi, le contact contre la chair du patient se fait plus ou moins délicatement selon la vitesse à laquelle le fourreau intérieur est entraîné hors du fourreau de support, et de manière générale, selon l'intensité de poussée exercée par les moyens élastiques sur le fourreau intérieur.

Cependant, l'impact du fourreau intérieur sur sa peau peut être désagréable pour le patient qui est généralement déjà impressionné par la piqûre.

La présente invention a pour but de proposer un dispositif de support de sécurité pour seringue dont la sortie en fin d'injection du fourreau intérieur hors du fourreau de support se fait en limitant l'appui entre le fourreau intérieur et la chair du patient.

Ce but est atteint grâce aux caractéristiques du système de support et du procédé suivant les revendications ci-jointes.

Ainsi, l'embout est adapté pour amortir une partie significative de l'énergie qui a pu être accumulée jusqu'au moment de la sortie du fourreau intérieur, en fin d'injection. Le matériau constituant l'embout, ainsi que sa géométrie peuvent être préférentiellement choisis en fonction des moyens de poussée retenus. En particulier, lorsque ces derniers comportent des moyens élastiques de raideur connue, le choix du matériau de l'embout est effectué en fonction de la dureté Shore adéquate pour amortir l'appui contre la peau du patient et pour que l'embout puisse encaisser une partie suffisante de l'énergie accumulée dans les moyens élastiques.

L'invention sera bien comprise et ses avantages apparaîtront mieux à la lecture de la description détaillée qui suit, de modes de réalisation représentés à titre d'exemples non limitatifs.

La description se réfère aux dessins annexés sur lesquels :
- la figure 1 est une vue en coupe d'un ensemble d'un dispositif de support de sécurité et d'une seringue en position escamotée d'attente,
- la figure 2A est une vue partielle en perspective prise selon la direction II indiquée sur la figure 1,
- la figure 2B est une coupe partielle selon les flèches IIB-IIB de la figure 1,
- la figure 3 est une vue en coupe de l'ensemble de la figure 1 en position active de protection,
- la figure 4 est une vue partielle en coupe de la partie avant du fourreau intérieur selon un autre mode de réalisation, et
- la figure 5 est une vue partielle en coupe de la partie avant du fourreau intérieur selon un autre mode de réalisation.

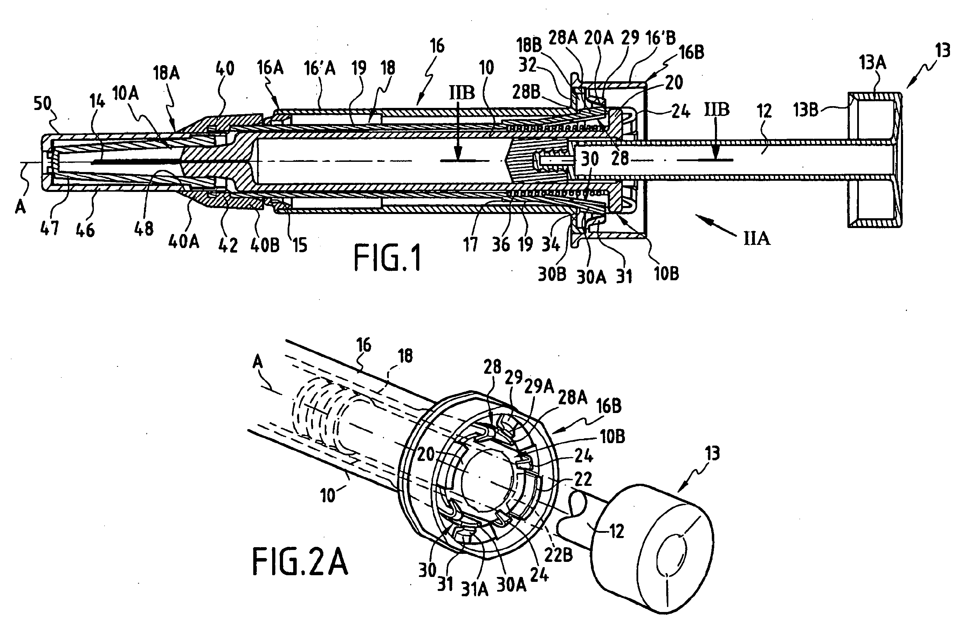

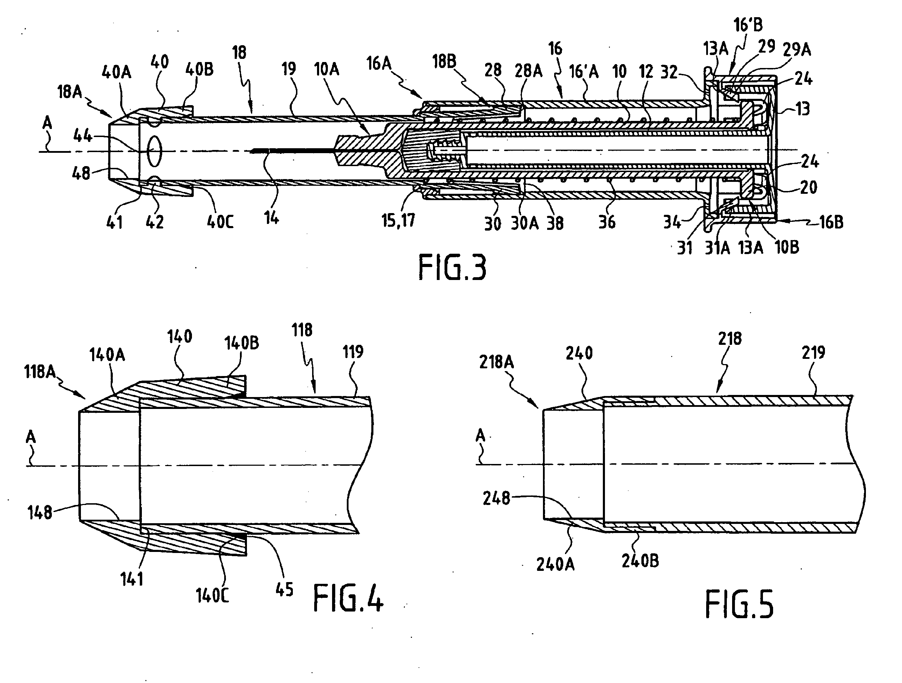

La seringue représentée sur les figures 1 à 4, comporte un corps 10 dans lequel un piston 12 peut coulisser entre une position d'attente d'injection, représentée sur la figure 1, et une position de fin d'injection, représentée sur les figures 3 et 4. A l'opposé du piston, une aiguille d'injection 14 est raccordée au corps 10. Le corps de la seringue 10 est préférentiellement en verre ou en plastique.

Le dispositif de support de sécurité pour cette seringue comporte un fourreau extérieur de support 16 et un fourreau intérieur 18 qui peut coulisser entre une position escamotée d'attente avant injection, représentée sur la figure 1, et une position active de protection après injection, représentée sur les figures 3 et 4. En position escamotée d'attente avant injection, le fourreau intérieur 18 est escamoté dans le fourreau extérieur 16, c'est-à-dire quasiment totalement rentré dans ce dernier, en étant disposé autour du corps 10 de la seringue et en étant retenu par rapport au fourreau extérieur 16 de telle sorte que l'aiguille 14 dépasse au-delà des extrémités avant 16A et 18A respectives des fourreaux intérieur 18 et de support 16.

Ces extrémités avant 16A et 18A sont bien entendu celles qui sont les plus proches du point d'injection, le sens vers l'avant étant le sens F de poussée du piston 12 dans le corps de seringue 10 pendant l'injection.

Le corps de seringue 10 est calé fixement par rapport au fourreau de support 16 par des moyens de calage appartenant à ce fourreau 16 et coopérant avec une collerette radiale 20 que présente l'extrémité arrière 10B du corps de seringue 10 (extrémité opposée à l'aiguille 14). La collerette 20 se trouve alors calée entre une butée comprenant en l'espèce deux épaulements 22B réalisés chacun à partir d'une portion de paroi axiale 22 rigides et des pattes d'encliquetage 24 ménagées à l'intérieur de ce fourreau de support 16 (par exemple, quatre pattes régulièrement réparties sur la périphérie interne de ce dernier). Ces deux portions de paroi axiale 22 sont diamétralement opposées sur le fourreau de support 16.

Lors de la mise en place du corps de seringue 10 dans le dispositif de support de sécurité, les pattes d'encliquetage 24, formées en arrière des épaulements 22B, s'écartent élastiquement pour permettre le passage de la collerette 20 du corps de seringue 10. Ainsi disposée, la collerette 20 est bien calée entre les épaulements 22B (dans le sens F de rentrée du piston) et les pattes d'encliquetage 24 (dans le sens opposé), de sorte que le corps de seringue 10 est maintenu fixement dans le fourreau de support 16 tout au long de l'injection et même pendant la sortie automatique en position active de protection du fourreau intérieur 18.

Comme on le voit plus en détails sur la figure 2A, la partie d'extrémité arrière 18B du fourreau intérieur 18 comporte en outre des languettes de retenue 28 et 30 axiales, dont les portions d'extrémités libres 28A et 30A présentant un épaulement 28B, respectivement 30B qui est respectivement accroché sur des surfaces de retenue 32 et 34 appartenant au fourreau de support 16. En l'espèce, ces surfaces de retenue 32 et 34 sont formées par deux épaulements respectifs ou par des parties d'un épaulement circulaire. En configuration active de retenue, les languettes de retenue 28 et 30 qui sont élastiques, ont une tendance naturelle à s'ouvrir en s'écartant élastiquement vers la périphérie du dispositif de support de sécurité.

Dans la configuration active de retenue, les languettes de retenue 28 et 30 du fourreau intérieur 18 retiennent ce dernier en position escamotée d'attente dans le fourreau de support 16. On pourrait, bien entendu, imaginer que ces languettes soient plutôt formées à la périphérie interne du fourreau extérieur 16 s'accrochant alors sur le fourreau intérieur 18 ou bien que ces languettes élastiques aient un sens d'accrochage inversé par rapport à celui que montre la figure 1 pour venir s'accrocher sur la collerette 20 du corps de seringue 10. Quelle que soit la variante envisagée, si comme c'est avantageusement le cas, on choisit que le piston 12 déclenche le fourreau intérieur 18, ces languettes de retenue 28 et 30 doivent pouvoir être décrochées lorsque le piston 12 de la seringue parvient dans une position de fin de course.

Les extrémités libres respectives 28A et 30A des languettes de retenue 28 et 30 du fourreau intérieur 16 viennent en regard de languettes d'activation 29 et 31 élastiques formées axialement sur le fourreau de support 16 qui sont susceptibles d'être sollicitées vers une configuration inactive dans lesquelles elles autorisent la sortie du fourreau intérieur 18 hors du fourreau de support 16. En l'espèce, ces languettes d'activation 29 et 31 peuvent être sollicitées par une partie de commande du piston 12.

Dans l'exemple représenté, la tête 13 du piston 12 présente cette partie de commande en forme de bride axiale 13A dirigée vers l'avant dont la périphérie interne 13B est inclinée, de telle sorte que, lorsque le piston 12 parvient en fin de course, cette périphérique interne 13B coopère avec les extrémités 29A et 31A des languettes d'activation 29 et 31, et sollicite ces dernières en les resserrant vers l'axe A. En se rapprochant de l'axe A, les languettes d'activation 29 et 31 sollicitent simultanément les portions d'extrémités 28A et 30A de sorte que languettes de retenue 28 et 30 se resserrent à leur tour vers l'axe A et libère leur épaulement 28B et 30B des surfaces de retenue respectives 32 et 34.

En fait, chacune des languettes d'activation 29 et 31 est disposée entre deux des quatre pattes d'encliquetage 24, tandis que chacune des portions axiales 22 est disposée entre les deux autres pattes d'encliquetage 24, de sorte qu'il existe une alternance du type languette d'activation / patte / portion axiale / patte sur toute la périphérie du fourreau de support 16.

Pour simplifier la réalisation du fourreau de support 16, ce dernier comporte comme dans l'exemple représenté, deux portions 16'A et 16'8, la portion 16'A étant de forme relativement simple, tandis que la portion 16'B à l'arrière du fourreau de support 16 plus complexe comporte l'ensemble de ces languettes 29, 31, de ces portions axiales 22 et de ces pattes 24. Les deux portions 16'A et 16'B sont maintenues l'une par rapport à l'autre par emmanchement ou tout autre moyen de coopération connu.

En fin de course du piston 12, le fourreau intérieur 18 se sépare du fourreau de support 16 par libération des languettes axiales de retenue 28 et 30 des surfaces de retenue respectives 32 et 34.

Des moyens de poussée, en l'espèce un ressort 36, favorisent la poussée vers l'avant (dans le sens F indiqué sur la figure 1) du fourreau intérieur 18, lorsque les languettes de retenue 28 et 30 sont libérées par pression des languettes d'activation 29 et 31 sollicitées par la tête 13 du piston 12. Ce ressort 36 est logé dans un espace annulaire ménagé entre le corps de seringue 10 et le fourreau intérieur 18.

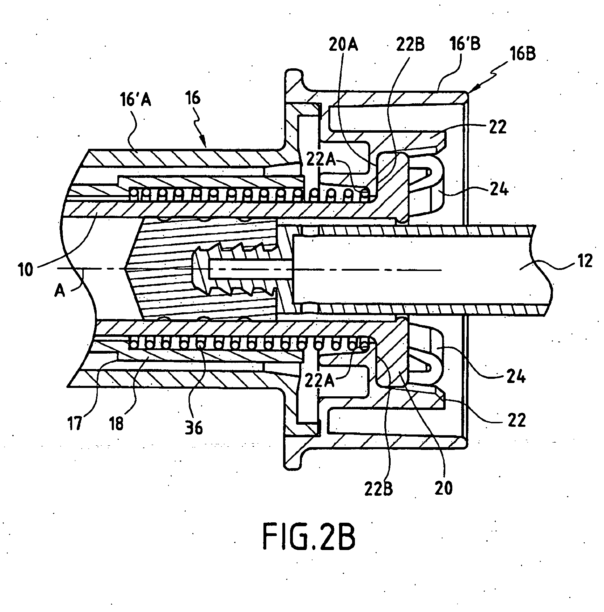

Comme on le voit sur la figure 2B, le fourreau intérieur 18 présente un épaulement 17 formé sur sa périphérie interne en retrait par rapport aux languettes de retenue 28 et 30. Cet épaulement 17 forme une surface d'appui pour le ressort 36. L'extrémité arrière du ressort 36 repose par ailleurs sur une face frontale 22A radiale de chacune des portions de paroi axiale 22.

En fait, les deux portions de paroi axiale 22 portent chacune un secteur radial en saillie vers l'axe A des fourreaux 16 et 18, les faces avant de ces secteurs forment les faces frontales 22A, tandis que leur face arrière forme les épaulements 22B.

Le fourreau intérieur 18 présente en outre une portion tubulaire 19 formée entre l'épaulement 17 et les languettes de retenue 28 et 30 qui s'étendent vers l'arrière du fourreau intérieur 18. Cette portion tubulaire présente un diamètre augmenté par rapport au diamètre du fourreau intérieur 18 et permet d'éviter le contact entre le ressort 36 et les languettes de retenue 28 et 30 et donc tout risque d'endommagement de ces dernières, par frottement par exemple.

L'espace annulaire ménagé pour le ressort 36 est donc limité par la surface d'appui formée par les faces frontales 22A, par l'épaulement 17, par la portion 19 tubulaire, et par les languettes de retenue 28 et 30. En position escamotée d'attente, le ressort 36 est ainsi maintenu précontraint entre ses surfaces d'appui formées d'une part, par les faces frontales 22A et d'autre part, par l'épaulement 17.

Après utilisation de la seringue, et en particulier en fin d'injection, comme illustré sur les figures 3 et 4, le piston 12 est en fin de course et sa tête 13 vient pratiquement en butée contre la collerette 20. Les languettes de retenue 28 et 30 ont été sollicitées par la tête 13, par l'intermédiaire des languettes d'activation 29 et 31, de sorte que les languettes de retenue 28 et 30 se sont décrochées du fourreau de support 16, en l'espèce des surfaces de retenue 32 et 34.

Le fourreau intérieur 18 n'étant plus retenu par les languettes de retenue 28 et 30 dans le fourreau de support 16, le ressort 36 a poussé le fourreau intérieur 18 vers l'avant de telle sorte que ce dernier dépasse largement au-delà de l'extrémité avant 16A du fourreau de support 16, sur une longueur telle qu'il forme une protection autour de l'aiguille 14 sur une longueur suffisante pour éviter les risques de contact d'un utilisateur avec l'aiguille 14.

Dans cette position active de protection, le fourreau intérieur 18 est calé vers l'avant par la venue en butée de son épaulement 17 avec un épaulement 15 formé intérieurement dans une région avant du fourreau extérieur 16. Des moyens de blocage permettent de bloquer le fourreau intérieur 18 dans cette position active de protection. Ces moyens de blocage permettent d'éviter qu'un simple appui sur l'extrémité libre 18A du fourreau intérieur 18 ne permette la rentrée de ce fourreau 18 dans le fourreau extérieur 16, auquel cas l'aiguille 14 pourrait devenir accessible par l'utilisateur.

Ces moyens de blocage comprennent en l'espèce un épaulement 38 formé dans le fourreau de support 16 et orienté à l'opposé de l'épaulement 15. Les extrémités libres 28A et 30A respectives des languettes axiales de retenue 28 et 30 sont aptes à coopérer avec cet épaulement 38 de sorte que le fourreau intérieur 18 se trouve calé entre le premier épaulement 15 l'empêchant d'aller vers l'avant dans le sens F, et le deuxième épaulement 38 l'empêchant de revenir vers l'arrière du dispositif de support de sécurité à l'opposé du déplacement de sortie du fourreau intérieur 18.

Le ressort 36 étant calé entre la face frontale 20A et l'épaulement 17, le dispositif de support de sécurité peut être livré préarmé avec ou sans seringue. La seringue est alors simplement mise en place dans le dispositif de support de sécurité par simple encliquetage de sa collerette 20 et calage de cette dernière entre les épaulements 22B et les pattes d'encliquetage 24 du fourreau de support 16.

Lorsqu'une seringue est mise en place dans le dispositif de support de sécurité, l'utilisateur manipule l'ensemble constitué par ce dispositif et par cette seringue en tenant le fourreau de support 16, par exemple entre l'index et le majeur, en les calant par exemple sur une collerette 16B formée à l'extrémité arrière de ce fourreau de support 16, et en manipulant le piston 12 par exemple avec le pouce.

La sortie du fourreau intérieur 18 se fait de manière automatique, sans intervention supplémentaire de l'utilisateur dès que le piston 12 atteint sa fin de course. Le choix de la raideur du ressort 36 permet de réguler cette sortie du fourreau intérieur 18 hors du fourreau de support et de limiter l'appui contre la chair du patient.

En outre, pour amortir l'appui dû à la percussion du fourreau intérieur 18 contre la peau du patient, le fourreau intérieur 18 comporte sur sa partie avant un embout 40 souple. L'extrémité libre de cet embout 40 forme l'extrémité libre 18A du fourreau intérieur 18.

Dans l'exemple représenté, le fourreau intérieur 18 est formé par deux parties de rigidité différentes : une première partie rigide 19 par laquelle le fourreau intérieur 18 coopère avec le fourreau de support 16, et une deuxième partie formée par l'embout 40 souple solidaire de la première partie 19.

Classiquement, le fourreau de support 16 et le fourreau intérieur 18 sont tubulaires et réalisés en plastique. Dans l'exemple représenté, l'intégralité de la première partie 19 est rigide, mais le fourreau intérieur 18 pourrait présenter des parties plus ou moins souples comportant des zones rigides qui lui permettent de coulisser sans se déformer dans le fourreau de support 16 et ainsi d'éviter tout risque d'arc-boutement dans ce dernier. Ces zones rigides pourraient, par exemple, être formées par des pattes rectilignes longitudinales qui s'étendraient le long de la première partie 19 du fourreau intérieur 18.

La deuxième partie du fourreau de support formé par l'embout 40 étant quant à elle destinée à amortir l'appui lors de la sortie du fourreau intérieur 18, elle est préférentiellement formée par un matériau plastique choisi parmi le PVC, les polyéthylènes, les élastomères ou le caoutchouc. Le matériau formant l'embout 40 présente avantageusement une dureté Shore comprise entre 30 Hs et 80 Hs, préférentiellement comprise entre 40 Hs et 50 Hs, pour assurer un bon amortissement, même en cas de sortie violente du fourreau intérieur 18, par exemple causé par une défaillance des moyens de retenue.

En outre, dans l'exemple représenté, l'embout 40 présente une épaisseur plus fine vers l'avant. En l'espèce, l'embout 40 présente une première partie 40B sensiblement tubulaire et une partie avant 40A sensiblement tronconique dont l'extrémité la plus fine est tournée vers l'avant du dispositif de sécurité.

Avec une telle conformation de l'embout 40, lors de la sortie du fourreau intérieur 18 hors du fourreau de support 16 et de l'arrivée du fourreau intérieur 18 contre la chair du patient, au moins une partie de l'énergie accumulée par le ressort 36 qui n'aurait pas été libérée par le déplacement du fourreau intérieur 18 dans le fourreau de support 16 serait encaissée par la déformation de l'embout 40.

Selon un premier mode de réalisation, illustré sur la figure 3, cet embout 40 est formé par une pièce additionnelle qui est fixée à la première partie du fourreau intérieur 18 par des moyens de fixation comportant des premiers moyens de fixation présentant au moins un organe en saillie et des deuxièmes moyens de fixation comportant au moins un organe en retrait.

Sur l'exemple représenté sur la figure 3, l'organe en saillie est en l'espèce un bourrelet 42 formé sur la périphérie interne de l'embout 40, et l'organe en retrait est en l'espèce formé par une encoche 44 pratiquée dans la première partie 19 du fourreau intérieur 18. De manière à faciliter la mise en place de l'embout 40, ce dernier présente à son extrémité arrière une ouverture 40C légèrement évasée pour faciliter l'introduction du fourreau intérieur 18. En outre, pour garantir le bon positionnement du fourreau intérieur 18 dans l'embout 40 au moment de l'encliquetage des bourrelets 42 dans les encoches 44, l'embout 40 présente un épaulement intérieur 41, contre lequel l'extrémité libre du fourreau intérieur 18 vient en butée.

On pourrait également imaginer que les bourrelets 42 soient formés sur la première partie 19 du fourreau intérieur 18 et que l'embout 40 présente des encoches 44 destinées à coopérer avec ces bourrelets 42 ou que cette première partie 19 et l'embout 40 présentent chacun un bourrelet et une encoche, le bourrelet de l'un coopérant avec l'encoche de l'autre. Dans ce mode de réalisation, toutes les formes possibles de l'organe en saillie et de l'organe en retrait permettant une coopération de l'embout 40 sur la première partie 19 du fourreau intérieur 18 peuvent être envisagées.

Selon un autre mode de réalisation, l'embout 140 est fixé à la première partie 119 du fourreau intérieur 118, par simple emmanchement en force de l'embout 140 sur l'extrémité libre de la première partie 119, comme illustré sur la figure 4. La pression exercée entre l'embout 140 et la première partie 119 suffit alors à maintenir l'embout 140 sur ce dernier.

Cet embout 140 peut en outre être fixé à la première partie 119 du fourreau intérieur 118 par des moyens 45, choisis parmi le collage, le surmoulage ou le soudage pour assurer son maintien en position.

Dans ce mode de réalisation, l'embout 140 se présente extérieurement de la même manière que l'embout 40 précédemment décrit, avec une partie tronconique 140A identique. De par l'absence de bourrelets, l'intérieur de la partie 140B est simplifiée en une forme cylindrique de diamètre intérieur correspondant au diamètre extérieur de la première partie 119 du fourreau intérieur 118, qui se prolonge jusqu'à l'épaulement 141. Le remplissage de l'ouverture évasée 140C (à l'opposée de l'avant 118A) par un adhésif 45 permet un bon maintien de l'embout 140 sur la première partie 119 du fourreau intérieur 118.

Selon un autre mode de réalisation illustré sur la figure 5, l'embout 240 peut être directement formé par moulage à l'extrémité de la première partie 219 du fourreau intérieur 118, ce dernier formant alors une seule et même pièce. Dans ce cas, deux matériaux, de rigidité différente, sont injectés dans un moule aux zones correspondantes, pour obtenir un fourreau intérieur 218 présentant une partie rigide 219 et une partie souple 240. L'embout 240 présente alors préférentiellement une partie arrière 240B dont la forme permet une bonne liaison entre les matériaux formant l'embout 240 et la première partie 219. La partie avant 218 se présente de manière amincie, comme pour les autres modes de réalisation, avec un contour intérieur 218 apte à coopérer avec un capuchon (non représenté).

Quelle que soit la variante envisagée, l'embout doit être suffisamment souple pour amortir les appuis lors de la percussion du fourreau intérieur contre la chair du patient, mais il est préférable d'éviter, pour des raisons d'hygiène, que l'embout puisse venir en contact avec une partie de l'aiguille. La déformation de l'embout ne doit pas pouvoir permettre à ce dernier de toucher l'aiguille, même lorsqu'une pression importante, due par exemple à un écrasement de l'embout, est exercée sur ce dernier. On pourra par exemple, selon la dureté du matériau, être amené à positionner l'extrémité libre de l'embout au ras de l'extrémité du corps de seringue proche de l'aiguille, en position escamotée d'attente.

L'ensemble formé par le dispositif de support de sécurité et par la seringue comporte en outre un capuchon amovible qui protège l'aiguille avant utilisation et en particulier lorsque le fourreau intérieur 18 est en position escamotée d'attente. Ce capuchon protège l'aiguille 14 de la seringue et coopère avec le corps de seringue 18 sur sa partie avant 10A. En l'espèce, le capuchon 46 représenté sur la figure 1 est en deux parties 46 et 47, disposées l'une autour de l'autre.

Dans la position escamotée d'attente du fourreau intérieur 18, l'embout 40 entoure sensiblement sans jeu le capuchon 46 de manière à protéger la seringue et l'aiguille 14 de toute contamination extérieure par liaison sensiblement étanche. En l'espèce, l'embout 40 présente un contour intérieur 48 de géométrie sensiblement identique à celle du contour extérieur du capuchon 46. Le capuchon 46 est fréquemment de forme tronconique évasée vers son ouverture, de sorte qu'il suffit de choisir un contour intérieur 48 de l'embout 40 de diamètre sensiblement égal au diamètre moyen du contour extérieur 40 du capuchon 46. Ainsi, l'embout 40 vient coopérer avec le capuchon 46 par légère déformation sur de l'embout 40 qui épouse le contour du capuchon 46. Le capuchon 46 amovible est retiré par l'utilisateur avant injection, en tirant simplement le capuchon 46 avec une main par exemple, tandis que l'autre main de l'utilisateur retient le dispositif de support de sécurité.

## Revendications

1. Un système de support de seringue de sécurité pour utilisation avec une seringue et comprenant, en combinaison,
a) un premier fourreau (18 ; 118) ;
b) un deuxième fourreau (16), les premier et deuxième fourreaux étant configurés pour se déplacer l'un par rapport à l'autre d'une position de départ à une position finale ; et
c) une fixation sur le deuxième fourreau (16), configurée pour engager une collerette (20) d'une seringue ayant un piston (12) avec une partie de tête (13) ; et
d) au moins deux languettes élastiques (28, 30) du premier fourreau
(18,118) configurées pour coopérer avec la partie de tête (13) du piston (12), de telle manière que, quand la tête (13) actionne les languettes (28,30), les premier et deuxième fourreaux se déplacent relativement l'un à l'autre de la position de départ à la position finale ; et
e) un ressort (36) de rappel des premier et deuxième fourreaux, configuré pour déplacer les fourreaux l'un par rapport à l'autre de la position de départ à la position finale ;
le système étant **caractérisé en ce qu'**il comprend aussi f) un embout souple (40 ; 140 ; 240) situé à une extrémité libre du premier fourreau (18 ; 118), ledit embout présentant une extrémité libre amincie (40A ; 140A).

2. Le système de support de seringue de sécurité suivant la revendication 1, comprenant en outre un dispositif d'arrêt en deux parties avec la première partie sur le premier fourreau (18 ; 118) et la deuxième partie sur le deuxième fourreau (16), dans lequel le dispositif d'arrêt est engagé quand les fourreaux sont dans la position finale, de manière à empêcher les fourreaux de se déplacer l'un par rapport à l'autre.

3. Le système de support de seringue de sécurité suivant une quelconque des revendications 1 ou 2, **caractérisé en outre en ce que** le premier fourreau (18,118) est configuré de manière à couvrir entièrement une aiguille (14) de la seringue quand les fourreaux sont dans leur position finale.

4. Le système de support de seringue de sécurité suivant une quelconque des revendications 1 à 3, **caractérisé en outre en ce que** les languettes (28,30) ont des crochets engageant le deuxième fourreau (16) dans la position de départ.

5. Le système de support de seringue de sécurité suivant la revendication 4, **caractérisé en outre en ce que** les crochets sont configurés pour se dégager du deuxième fourreau quand la tête (13) actionne les languettes (28,30).

6. Le système de support de seringue de sécurité suivant une quelconque des revendications 4 ou 5, **caractérisé en outre en ce que** les fourreaux sont aptes à se déplacer l'un par rapport à l'autre quand les crochets sont dégagés du deuxième fourreau (16).

7. Le système de support de seringue de sécurité suivant une quelconque des revendications 1 à 6, **caractérisé en ce qu'**un des fourreaux a une collerette (16B) placée à une extrémité proximale.

8. Le système de support de seringue de sécurité suivant une quelconque des revendications 1 à 7, comprenant en outre deux ou plus dispositifs d'arrêt en deux parties.

9. Un système de support de seringue de sécurité suivant la revendication 1, dans lequel :
au moins deux languettes élastiques (29,31) se prolongent axialement à partir d'un des fourreaux dans une direction proximale, chaque languette se terminant par une rampe ayant une première surface d'appui configurée de manière à engager une deuxième surface d'appui (13B) sur la périphérie intérieure d'une partie de tête (13) d'un piston (12) d'une seringue en fin de course du piston (12) pour causer une flexion radiale des languettes permettant le déplacement relatif des fourreaux, l'un par rapport à l'autre, de la position de départ à la position finale.

10. Un système de support de seringue de sécurité suivant la revendication 1, dans lequel
lesdites languettes élastiques (28,30) se prolongent axialement à partir d'un des fourreaux dans une direction proximale pour engager l'autre fourreau de manière à bloquer les fourreaux dans la position de départ, les languettes (28,30) étant configurées pour être actionnées par une partie de tête (13) d'un piston (12) d'une seringue en fin de course du piston (12) de manière à causer une flexion radiale des languettes permettant le déplacement relatif des fourreaux, l'un par rapport à l'autre, de la position de départ à la position finale ; le système de support de seringue de sécurité comprenant aussi un ressort (36) de rappel des premier et deuxième fourreaux configuré pour déplacer les fourreaux l'un par rapport à l'autre de la position de départ à la position finale.

11. Un procédé pour protéger un utilisateur d'une seringue, utilisant un système de support de seringue de sécurité comprenant un premier et un deuxième fourreaux configurés pour se déplacer l'un par rapport à l'autre d'une position de départ à une position finale suite à l'activation d'au moins deux languettes (28,30) élastiques prolongeant un premier des fourreaux dans une direction proximale, le premier fourreau (18 ; 118) présentant une portion d'extrémité libre formée par un embout souple (40 ; 140 ; 240) présentant une extrémité libre amincie (40A ; 140A), et lesdits fourreaux contenant une seringue avec une aiguille (14) préalablement attachée, le procédé comprenant :
a) l'actionnement des languettes (28,30) par la partie de tête (13) d'un piston (12) de la seringue en fin de course, fléchissant les languettes (28,30) pour dégager des crochets engagés au deuxième fourreau (16) ;
b) le déplacement relatif des fourreaux l'un par rapport à l'autre vers leur position finale, dans laquelle l'aiguille est sensiblement couverte par le premier fourreau (18,118).

## Patentansprüche

1. Sicherheitsspritzentragsystem zur Verwendung mit einer Spritze und umfassend, in Kombination,
a) eine erste Hülse (18; 118);
b) eine zweite Hülse (16), wobei die erste und die zweite Hülse dazu ausgebildet sind, sich von einer Ausgangsstellung in eine Endstellung zueinander zu bewegen; und
c) eine Befestigung an der zweiten Hülse (16), die dazu ausgebildet ist, einen Kragen (20) einer Spritze, welche einen Kolben (12) mit einem Kopfteil (13) aufweist, zu belegen; und
d) wenigstens zwei elastische Zungen (28, 30) der ersten Hülse (18, 118), die dazu ausgebildet sind, mit dem Kopfteil (13) des Kolbens (12) derart zusammenzuwirken, dass wenn der Kopf (13) die Zungen (28, 30) betätigt, die erste und die zweite Hülse sich von der Ausgangsstellung in die Endstellung relativ zueinander bewegen; und
e) eine Feder (36) zum Rückstellen der ersten und der zweiten Hülse, die dazu ausgebildet ist, die Hülsen von der Ausgangsstellung in die Endstellung zueinander zu bewegen;
wobei das System **dadurch gekennzeichnet ist, dass** es auch umfasst f) ein flexibles Ansatzstück (40; 140; 240), das an einem freien Ende der ersten Hülse (18; 118) gelegen ist, wobei das Ansatzstück ein verjüngtes freies Ende (40A; 140A) aufweist.

2. Sicherheitsspritzentragsystem nach Anspruch 1, ferner umfassend eine zweiteilige Sperrvorrichtung, mit dem ersten Teil an der ersten Hülse (18; 118) und dem zweiten Teil an der zweiten Hülse (16), wobei die Sperrvorrichtung eingerückt ist, wenn die Hülsen sich in der Endstellung befinden, um die Hülsen daran zu hindern, sich zueinander zu bewegen.

3. Sicherheitsspritzentragsystem nach einem der Ansprüche 1 oder 2, ferner **dadurch gekennzeichnet, dass** die erste Hülse (18, 118) derart ausgebildet ist, dass sie eine Nadel (14) der Spritze vollständig bedeckt, wenn die Hülsen sich in ihrer Endstellung befinden.

4. Sicherheitsspritzentragsystem nach einem der Ansprüche 1 bis 3, ferner **dadurch gekennzeichnet, dass** die Zungen (28, 30) Haken haben, die die zweite Hülse (16) in der Ausgangsstellung belegen.

5. Sicherheitsspritzentragsystem nach Anspruch 4, ferner **dadurch gekennzeichnet, dass** die Haken dazu ausgebildet sind, sich von der zweiten Hülse zu lösen, wenn der Kopf (13) die Zungen (28, 30) betätigt.

6. Sicherheitsspritzentragsystem nach einem der Ansprüche 4 oder 5, ferner **dadurch gekennzeichnet, dass** die Hülsen geeignet sind, sich zueinander zu bewegen, wenn die Haken von der zweiten Hülse (16) gelöst sind.

7. Sicherheitsspritzentragsystem nach einem der Ansprüche 1 bis 6, **dadurch gekennzeichnet, dass** eine der Hülsen einen Kragen (16B), welcher an einem proximalen Ende angeordnet ist, umfasst.

8. Sicherheitsspritzentragsystem nach einem der Ansprüche 1 bis 7, ferner umfassend zwei oder mehr zweiteilige Sperrvorrichtungen.

9. Sicherheitsspritzentragsystem nach Anspruch 1, wobei:
wenigstens zwei elastische Zungen (29, 31) sich von einer der Hülsen ausgehend in eine proximale Richtung axial fortsetzen, wobei jede Zunge mit einer Rampe abschließt, welche eine erste Anlagefläche aufweist, die ausgebildet ist, um eine zweite Anlagefläche (13B) am Innenumfang eines Kopfteils (13) eines Kolbens (12) einer Spritze in Endstellung des Kolbens (12) zu belegen, um eine radiale Biegung der Zungen zu bewirken, welche die Relativbewegung der Hülsen zueinander von der Ausgangsstellung in die Endstellung ermöglicht.

10. Sicherheitsspritzentragsystem nach Anspruch 1, wobei:
die elastischen Zungen (28, 30) sich von einer der Hülsen ausgehend in eine proximale Richtung axial fortsetzen, um die andere Hülse derart zu belegen, dass die Hülsen in der Ausgangsstellung blockiert werden, wobei die Zungen (28, 30) dazu ausgebildet sind, durch einen Kopfteil (13) eines Kolbens (12) einer Spritze in Endstellung des Kolbens (12) betätigt zu werden, um eine radiale Biegung der Zungen zu bewirken, welche die Relativbewegung der Hülsen zueinander von der Ausgangsstellung in die Endstellung ermöglicht, wobei das Sicherheitsspritzentragsystem auch eine Feder (36) zum Rückstellen der ersten und der zweiten Hülse umfasst, die dazu ausgebildet ist, die Hülsen von der Ausgangsstellung in die Endstellung zueinander zu bewegen.

11. Verfahren zum Schützen eines Benutzers einer Spritze, das ein Sicherheitsspritzentragsystem verwendet, welches eine erste und eine zweite Hülse umfasst, die dazu ausgebildet sind, sich in Folge der Aktivierung von wenigstens zwei elastischen Zungen (28, 30), die eine erste der Hülsen in einer proximalen Richtung fortsetzen, von einer Ausgangsstellung in eine Endstellung zueinander zu bewegen, wobei die erste Hülse (18; 118) einen freien Endabschnitt aufweist, der von einem flexiblen Ansatzstück (40; 140; 240) gebildet ist, das ein verjüngtes freies Ende (40A; 140A) aufweist, und wobei die Hülsen eine Spritze mit einer zuvor befestigten Nadel (14) enthalten, wobei das Verfahren umfasst:
a) das Betätigen der Zungen (28, 30) durch den Kopfteil (13) eines Kolbens (12) der Spritze in Endstellung, das die Zungen (28, 30) biegt, um Haken, welche an der zweiten Hülse (16) in Eingriff sind, zu lösen;
b) das Relativbewegen der Hülsen zueinander in Richtung ihrer Endstellung, in der die Nadel durch die erste Hülse (18, 118) im Wesentlichen bedeckt ist.

## Claims

1. A safe syringe support system for use with a syringe, said safe syringe support system comprising and being combined with:
a) a first sheath (18; 118);
b) a second sheath (16), the first and second sheaths being configured to move in relation to each other from an initial position to a final position; and
c) fixing means on the second sheath (16) configured to engage a collar (20) of a syringe having a piston (12) that is provided with a head portion (13);
d) at least two resilient tongues (28, 30) of the first sheath (18, 118) configured to cooperate with the head portion (13) of the piston (12) in such a way that, when the head (13) actuates the tongues (28, 30), the first and second sheaths move in relation to each other from the initial position to the final position; and
e) a return spring (36) for returning the first and second sheaths that is configured to move the sheaths in relation to each other from the initial position to the final position;
the system being **characterized in that** it further comprises f) a flexible end-piece (40; 140; 240) that is situated at a free end of the first sheath (18; 118), with said end-piece having a tapering free end (40A; 140A).

2. The safe syringe support system according to claim 1, further comprising a two-part stopping device with the first part on the first sheath (18; 118) and the second part on the second sheath (16), wherein the stopping device is initiated when the sheaths are in the final position, so as to prevent the sheaths from moving in relation to each other.

3. The safe syringe support system according to claim 1 or claim 2, further **characterized in that** the first sheath (18; 118) is configured so as to fully cover a needle (14) of the syringe when the sheaths are in their final positions.

4. The safe syringe support system according to any one of claims 1 to 3, further **characterized in that** the tongues (28, 30) have hooks that engage the second sheath (16) in the initial position.

5. The safe syringe support system according to claim 4, further **characterized in that** the hooks are configured to be released from the second sheath when the head (13) actuates the tongues (28, 30).

6. The safe syringe support system according to claim 4 or claim 5, further **characterized in that** the sheaths are suitable for moving in relation to each other when the hooks are released from the second sheath (16).

7. The safe syringe support system according to any one of claims 1 to 6, **characterized in that** one of the sheaths has a collar (16B) that is provided on one proximal end.

8. The safe syringe support system according to any one of claims 1 to 7, further comprising two or more two-part stopping devices.

9. A safe syringe support system according to claim 1, wherein:
at least two resilient tongues (29, 31) axially extend from one of the sheaths into a proximal direction, each tongue terminating by a ramp having a first bearing surface that is configured so as to engage a second bearing surface (13B) on the inner periphery of a head portion (13) of a piston (12) of a syringe, at the end of the stroke of the piston (12), so as to cause radial bending of the tongues, whereby the sheaths are allowed relative movement in relation to each other from the initial position to the final position.

10. A safe syringe support system according to claim 1, wherein:
said resilient tongues (28, 30) axially extend from one of the sheaths into a proximal direction so as to engage the other sheath in order to lock the sheaths in the initial position, the tongues (28, 30) being configured to be actuated by a head portion (13) of a piston (12) of a syringe, at the end of the stroke of the piston (12), so as to cause radial bending of the tongues, whereby the sheaths are allowed relative movement in relation to each other from the initial position to the final position; the safe syringe support system likewise comprising a return spring (36) for returning the first and second sheaths, said spring being configured so as to move the sheaths in relation to each other from the initial position to the final position.

11. A method for protecting a user against a syringe, the method using a safe syringe support system comprising first and second sheaths configured to move in relation to each other from an initial position to a final position following the actuation of at least two resilient tongues (28, 30) extending a first sheath into a proximal direction, the first sheath (18; 118) having a free end portion formed by a flexible end-piece (40A; 140A) and said sheaths containing a syringe with a needle (14) previously attached thereto, the method comprising:
a) the actuation of the tongues (28, 30) by means of the head portion (13) of a piston (12) of the end-of-stroke syringe which bends the tongues (28, 30) in order to release the hooks engaged in the second sheath (16); and
b) the relative movement of the sheaths in relation to each other towards their final position wherein the needle is substantially covered by the first sheath (18, 118).
